# EUROPÄISCHE PATENTSCHRIFT

(11) **EP 2 853 242 B1**
(45) Veröffentlichungstag und Bekanntmachung des Hinweises auf die Patenterteilung: **31.01.2018**
(21) Anmeldenummer: 14183311.1
(22) Anmeldetag: 03.09.2014
(51) Int. Cl.: A61F 2/38, A61F 2/32, A61F 2/36, A61F 2/30

(54) **Modulares Gelenkspacer-System**
Modular articulated spacer system
Système espaceur à articulation modulaire

(30) Priorität: 27.09.2013 DE 102013219656
(43) Veröffentlichungstag der Anmeldung: 01.04.2015
(73) Patentinhaber: Heraeus Medical GmbH, 61273 Wehrheim (DE)
(72) Erfinder: Dr. Vogt, Sebastian, 99092 Erfurt (DE)
(74) Vertreter: Schultheiss & Sterzel Patentanwälte PartG mbB

(56) Entgegenhaltungen:
- EP-A1- 1 166 724
- EP-A2- 0 393 608
- WO-A1-98/51240
- WO-A1-2010/015877
- US-A1- 2013 072 896

## Beschreibung

Die Erfindung betrifft ein modulares Gelenkspacer-System zum Zusammenschrauben eines Gelenkspacers, der zum zeitweisen Ersetzen eines künstlichen Gelenks vorgesehen ist, wobei das Gelenkspacer-System zumindest zwei über eine Schraubverbindung verbindbare Module aufweist, wobei ein erstes Modul des Gelenkspacer-Systems eine Oberfläche zur Ausbildung einer Gleitfläche des Gelenks des Gelenkspacers aufweist und ein zweites Modul des Gelenkspacer-Systems einen Schaft zur Verbindung mit einem Knochen aufweist.

Die Erfindung betrifft auch ein Verfahren zum Aufbau eines Gelenkspacers mit einem solchen Gelenkspacer-System und die Verwendung eines Gelenkspacers hergestellt aus einem solchen Gelenkspacer-System.

Gelenkendoprothesen haben gegenwärtig eine Standzeit von mehreren Jahren, zum Beispiel bei zementierten Hüftgelenkprothesen im Durchschnitt größer als zehn bis fünfzehn Jahre. Es gibt jedoch unerwünschte Lockerungen der Gelenkendoprothesen, die vor dem Erreichen der üblichen Standzeiten auftreten. Es wird dabei die septische und die aseptische Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen.

Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich bei der Behandlung mit sehr hohen Kosten verbunden ist. Sowohl bei der aseptischen als auch der septischen Lockerung wird üblicherweise eine Revision vorgenommen. Man unterscheidet einzeitige und zweizeitige Revisionen. Bei septischen Lockerungen werden sehr häufig zweizeitige Revisionen vorgenommen.

Bei der zweizeitigen Revision wird in einer ersten Operation (OP) die infizierte Gelenkendoprothese entfernt, es erfolgt ein Debridement (ein Entfernen des infizierten Gewebes) und anschließend wird ein temporärer Platzhalter, ein so genannter Spacer, eingesetzt. Dieser Spacer füllt für einige Wochen den Raum der zuvor revidierten Endoprothese aus, bis die vorliegende Infektion abgeklungen ist. Diese Platzhalterfunktion ist sehr wichtig, um eine Atrophie der Muskulatur in dieser Zeit wirksam zu verhindern und um eine Stabilisierung der Resektionssituation zu erreichen.

Man unterscheidet nichtartikulierende und artikulierende Spacer. Artikulierende Spacer, die im Folgenden als Gelenkspacer bezeichnet werden, bilden die Gelenkfunktion nach und erlauben eine gewisse Beweglichkeit der betroffenen Gliedmaßen. Es ist dadurch möglich, die Patienten frühzeitig zu mobilisieren. Gelenkspacer werden daher derzeit besonders gerne eingesetzt. Als Gelenkspacer kommen vor allem Hüftspacer, Kniespacer und Ellenbogenspacer zum Einsatz. Der Spacer wird in einer zweiten OP entfernt, es wird nochmals debridiert und dann wird eine zementierte oder auch zementfreie Revisions-Gelenkendoprothese implantiert.

Die Verwendung von Spacern geht ursprünglich auf Hovelius und Josefsson zurück (Hovelius L, Josefsson G (1979), "An alternative method for exchange operation of infected arthroplasty", Acta Orthop. Scand. 50: 93-96). Weitere frühe Arbeiten zu Spacern stammen von Younger (Younger AS, Duncan CP, Masri BA, McGraw RW (1997), "The outcome of two-stage arthroplasty using a custom-made interval spacer to treat the infected hip", J. Arthroplasty 12: 615-623), Jones (Jones WA, Wroblewski BM (1989), "Salvage of failed total knee arthroplasty: the 'beefburger' procedure", J. Bone Joint Surg. Br. 71: 856-857.) und Cohen (Cohen JC, Hozack WJ, Cuckler JM, Booth RE Jr (1988), "Two-stage reimplantation of septic total knee arthroplasty, Report of three cases using an antibiotic-PMMA spacer block", J. Arthroplasty 3: 369-377). Von McPherson stammt die Konzeption, dass Spacer ausschließlich aus Knochenzement hergestellt werden können (McPherson EJ, Lewonowski K, Dorr LD (1995), "Techniques in arthroplasty. Use of an articulated PMMA spacer in the infected total knee arthroplasty", J. Arthroplasty 10: 87-89).

Gegenwärtig gibt es auf dem Markt von einer Reihe von vorgefertigten artikulierenden Hüft- und Kniespacern auf Basis von Polymethylmethacrylat-Knochenzement, der mit Gentamicin und/oder Vancomycin dotiert ist. Diese Spacer gibt es in verschieden Größen, die weitgehend das Spektrum der Variabilität der anatomischen Verhältnisse hinreichend abdecken. Wünschenswert wäre es jedoch, wenn der medizinische Anwender während der Revisions-OP, je nach den beim jeweiligen Patienten vorgefundenen anatomischen Verhältnissen sowohl den Abstand zwischen dem Spacerkopf und dem Schaft als auch den CCD-Winkel (Centrum-Collum-Diaphysen-Winkel) individuell einstellen könnte.

In der FR 2 948 012 A1 ist ein Hüftimplantat beschrieben, bei dem ein Kopf des Implantats auf einen Schaft des Implantats aufgesteckt werden kann, wobei der Kopf Tanks enthält, die vor der Implantation mit antibiotischen Lösungen befüllt werden können. Nachteilig ist hieran, dass zum einen eine dauerhafte Fixierung des Kopfs nicht näher beschrieben ist und dass der Winkel des Kopfs nicht einstellbar ist.

In der US 2013/072 896 A1 wird ein gattungsgemäßes modulares Hüftspacersystem vorgeschlagen, bei dem der Abstand zwischen einem Spacerkopf und einem Spacerschaft durch unterschiedlich weites Aufschrauben des Spacerkopfs auf ein Gewinde am Spacerschaft eingestellt werden kann. Die Schraubverbindung ist dabei mit einer antibiotisch ausgerüsteten Beschichtung versehen. Eine dauerhafte, verdrehsichere Fixierung erfolgt später durch Zementierung mit Polymethylmethacrylat-Knochenzement (PMMA-Knochenzement). Auch die WO 2010/015877 A1 offenbart ein Spacersystem, bei dem die Teile eines Spacers zusammengeschraubt werden und mit einem selbsthärtenden flüssigen Zement fixiert werden.

Das hat den Nachteil, dass es bis zum Aushärten des Knochenzements zu einer Veränderung der Anordnung kommen kann. Ferner ist es nachteilig, dass das Arbeiten mit dem Knochenzement während einer OP aufwendig ist, sich ablösende Knochenzementreste den OP-Raum kontaminieren können und einige Werkzeuge, wie beispielsweise ein Spachtel für die Anwendung des Knochenzements bereitgestellt werden müssen. Zudem ist auch bei diesem System der Winkel des Spacerkopfs gegenüber dem Spacerschaft nicht einstellbar.

Die Aufgabe der Erfindung besteht also darin, die Nachteile des Stands der Technik zu überwinden. Insbesondere soll ein einfach anzuwendendes Gelenkspacer-System bereitgestellt werden, das im oft hektischen OP-Betrieb möglichst unaufwendig und unanfällig für Fehlbedienungen ist. Ferner soll ein modular aufgebautes Gelenkspacer-System entwickelt werden, bei dem der Abstand zwischen dem Spacerschaft und dem Spacerkopf variabel eingestellt werden kann und bei dem zusätzlich der CCD-Winkel zwischen Spacerschaft und Spacerkopf variiert werden kann. Das Gelenkspacer-System soll insbesondere für Hüftspacer-Systeme geeignet sein. Eine wesentliche Aufgabe der Erfindung besteht weiterhin darin, dass die Module des modularen Gelenkspacer-Systems nach erfolgter Patienten-adaptierter Einstellung des Abstands zwischen Spacerschaft und Spacerkopf und dem CCD-Winkel durch den medizinischen Anwender in einfachster Weise ohne Verwendung von Polymethylmethacrylat-Knochenzement irreversibel, verdrehsicher fixiert werden können. Das bedeutet, dem medizinischen Anwender soll ein modulares Gelenkspacer-System zur Verfügung gestellt werden, das mit kürzestem Zeit- und Arbeitsaufwand entsprechend den anatomischen Patientenverhältnissen formstabil, irreversibel ohne Verwendung von Spezialwerkzeug fixiert werden kann. Gleichzeitig soll das Gelenkspacer-System kostengünstig sein.

Die Aufgaben der Erfindung werden gelöst durch ein modulares Gelenkspacer-System zum Zusammenschrauben eines Gelenkspacers, der zum zeitweisen Ersetzen eines künstlichen Gelenks vorgesehen ist, wobei das Gelenkspacer-System zumindest zwei über eine Schraubverbindung verbindbare Module aufweist, wobei ein erstes Modul des Gelenkspacer-Systems eine Oberfläche zur Ausbildung einer Gleitfläche des Gelenks des Gelenkspacers aufweist und ein zweites Modul des Gelenkspacer-Systems einen Schaft zur Verbindung mit einem Knochen aufweist, bei dem die über die Schraubverbindung verbindbaren Module für jede Schraubverbindung zumindest eine Schraubensicherung aufweisen, wobei die zumindest eine Schraubensicherung mit einem der Module verbunden ist oder einteilig mit einem Modul ausgeführt ist, wobei zu jeder Schraubverbindung zwei Schraubensicherungsteile vorgesehen sind, die fest mit den beiden über die Schraubverbindung zu verbindenden Modulen verbunden sind oder die einteilig mit den beiden über die Schraubverbindung zu verbindenden Modulen ausgeführt sind und die beim Zusammenschrauben der Module derart ineinander greifen, dass sie einem Lösen der Schraubverbindung entgegenwirken, bevorzugt ein Lösen der Schraubverbindung verhindern.

Die Schraubensicherungen können beispielsweise durch Keilsicherungsscheiben realisiert werden, die bevorzugt einteilig mit den Modulen aufgebaut sind. Alternativ können an den Modulen auch Sperrzähne, Sicherungsscheiben mit Rippen, Federscheiben oder Schnorrscheiben an den Elementen als Schraubensicherung vorgesehen sein. Erfindungsgemäß bevorzugt werden jedoch Zahnscheiben als Schraubensicherung eingesetzt. Erfindungsgemäss ist die Schraubensicherung fest mit einem der Module verbunden ist oder einteilig mit einem Modul ausgeführt ist. Theoretisch kann es ausreichen, wenn die Schraubensicherung an das Modul geklebt oder mit diesem verschweißt ist, um eine Verbindung mit dem Modul zu realisieren.

Die mit dem Gelenkspacer-System aufgebauten Gelenkspacer können insbesondere Hüftspacer, Kniespacer oder Ellenbogenspacer sein. Demzufolge kann es sich bei dem Gelenkspacer-System erfindungsgemäß um ein Hüftspacer-System, Kniespacer-System oder Ellenbogenspacer-System handeln, wobei Hüftspacer-Systeme im Rahmen der vorliegenden Erfindung besonders bevorzugt sind, da sich hier die Variabilität des erfindungsgemäßen Gelenkspacer-Systems besonders vorteilhaft auswirkt. Bei einem künstlichen Hüftgelenk und damit bei einem Hüftgelenkspacer können geringe Fehlstellungen oder Abweichungen von der optimalen Form schnell zu Rückenschmerzen und anderen Leiden führen, so dass eine präzise Behandlung des Patienten, wie sie durch das erfindungsgemäße Gelenkspacer-System möglich ist, besonders wichtig ist.

Die Elemente der Schraubverbindung sind erfindungsgemäß bevorzugt eine Gewindestange mit einem Außengewinde und eine Überwurfmutter mit einem Innengewinde.

Mit der Erfindung wird auch ein Gelenkspacer-System vorgeschlagen, das zumindest drei über Schraubverbindungen verbindbare Module aufweist und zumindest zwei Module wenigstens eine Schraubensicherung aufweisen.

Hierdurch können der Gelenkkopf, der Schaft und der Winkel zwischen den beiden über separate Module, nämlich ein Gelenkkopfmodul, ein Schaftmodul und ein Adaptermodul unabhängig voneinander eingestellt werden. Durch diese Maßnahme erhält man also besonders variabel einsetzbare Gelenkspacer-Systeme.

Ferner ist erfindungsgemäß vorgesehen, dass zu jeder Schraubverbindung zwei Schraubensicherungsteile vorgesehen sind, die fest mit den beiden über die Schraubverbindung zu verbindenden Module verbunden sind oder die einteilig mit den beiden über die Schraubverbindung zu verbindenden Module ausgeführt sind und die beim Zusammenschrauben der Module derart ineinander greifen, dass die einem Lösen der Schraubverbindung entgegenwirken, bevorzugt ein Lösen der Schraubverbindung verhindern.

Durch das Vorsehen von zwei Schraubensicherungsteilen, die ineinander greifen, kann eine besonders stabile Verbindung hergestellt werden, insbesondere auch aus Materialien gleicher Härte. Lösungen mit unterschiedlichen Materialien sind weniger bevorzugt, da ein metallischer Abrieb durch mechanische Verformung einer Komponente bei einer Verhakung der Schraubensicherungsteile ineinander vermieden werden soll. Ebenso sollen Klebstoffverbindungen vermieden werden, da diese meist nicht ausreichend biokompatibel sind und sich störend auf den Heilungsprozess auswirken könnten.

Mit einer Weiterbildung der Erfindung wird vorgeschlagen, dass jede Schraubverbindung ein Innengewinde und ein zum Innengewinde passendes Außengewinde aufweist, und zwei über eine Schraubverbindung miteinander verbindbare Module an den Enden, die im miteinander verbundenen Zustand zueinander weisen, jeweils das Innengewinde und das Außengewinde dieser Schraubverbindung ausgebildet ist, so dass die Schraubverbindung ein Teil der beiden Module ist.

Dies stellt die einfachste Form einer Schraubverbindung dar.

Dabei kann vorgesehen sein, dass an jeweils einem der miteinander verbindbaren Module am Ende des Außengewindes oder am Anfang des Innengewindes die Schraubensicherung vorgesehen ist oder an beiden miteinander verbindbaren Modulen am Ende des Außengewindes oder am Anfang des Innengewindes ein Schraubensicherungsteil vorgesehen ist.

Hierdurch kann sichergestellt werden, dass die Schraubensicherungen erst beim vollständigen Aneinanderschrauben der Module greifen und die Module unlösbar miteinander verbinden.

Des Weiteren kann dabei vorgesehen sein, dass die Module jeweils einen Metallkern aufweisen, wobei der Metallkern aus einem der zueinander weisenden Enden der miteinander verbindbaren Module herausragt und dort das Außengewinde bildet und in dem Metallkern des anderen Moduls, der miteinander verbindbaren Module ein Loch mit dem Innengewinde vorgesehen ist, so dass das Außengewinde und das Innengewinde eine Schraubverbindung bilden. Dabei kann bevorzugt vorgesehen sein, dass auch die Schraubensicherung durch wenigstens einen der Metallkerne gebildet ist, besonders bevorzugt die Schraubensicherung durch die beiden Metallkerne gebildet ist.

Die Metallkerne haben eine für den medizinischen Zweck besonders gut geeignete Stabilität und führen dazu, dass sich ein stabiler Gelenkspacer aus dem Gelenkspacer-System aufbauen lässt. Zudem sind die Metalle der Metallkerne besonders gut zur einteiligen Ausbildung der Gewinde der Schraubverbindung und zur Ausbildung geeigneter Schraubensicherungen geeignet.

Es kann dabei erfindungsgemäß bevorzugt vorgesehen sein, dass die Metallkerne aus einem Chrom-Kobalt-Stahl oder einem V4A-Stahl bestehen.

Die einteilige Ausführung stellt sicher, dass die Verbindung eines Moduls zu dem ihm zugehörigen Schraubensicherungsteil besonders stabil ist. Zudem kann so die Anzahl der notwendigen Teile reduziert werden, was eine besonders einfache Anwendbarkeit des Gelenkspacer-Systems bewirkt.

Mit einer Weiterentwicklung der Erfindung wird vorgeschlagen, dass die zumindest eine Schraubensicherung zwei Zahnscheiben oder eine zweiteilige Keilsicherung ist.

Diese Schraubensicherungen sind besonders vorteilhaft, da hiermit auch ohne plastische Verformung und ohne Klebstoffe eine stabile Rückdrehsicherung beziehungsweise Schraubensicherung möglich ist.

Dabei kann vorgesehen sein, dass die Flanken der Zähne der Zahnscheiben oder der Keile der Keilsicherungen entgegen der Drehrichtung der Elemente der zumindest einen Schraubverbindung geneigt sind und die Höhe der Zähne der Zahnscheiben oder der Keile der Keilsicherungen größer ist, als die Ganghöhe der Gewinde der Elemente der zumindest einen Schraubverbindung.

Hierdurch wird eine einfache Verschraubung ermöglicht und gleichzeitig eine zuverlässige Sperrung bei einem Versuch des Lösens der Schraubverbindung erreicht.

Es kann auch vorgesehen sein, dass die Keilsicherungen auf der Außenseite Radialrippen und auf der Innenseite Keilflächen aufweisen, deren Steigung größer als die Gewindesteigung ist. Aufgrund der Radialrippen kommt es beim Festziehen zu einem Formschluss. Das Keilsicherungspaar sitzt dann fest an seinem Platz und Bewegungen sind nur noch zwischen den Keilflächen möglich. Schon bei geringer Drehung in Löserichtung erfolgt aufgrund der Keilwirkung eine Erhöhung der Klemmkraft und die Schraubverbindung sichert sich über die Schraubensicherung somit selbst.

Es kann auch vorgesehen sein, dass jede Schraubverbindung und jede Schraubensicherung aus einem biokompatiblen Material besteht.

Biokompatible Materialien sind zur Verwendung im menschlichen Körper geeignet.

Gemäß einer vorteilhaften Weiterbildung der Erfindung kann vorgesehen sein, dass zwischen den Modulen Abstandhalter in Form von Zahnscheiben mit beidseitiger Zahnung oder Keilsicherungen mit beidseitigen Keilen vorgesehen sind, wobei bevorzugt die Abstandhalter aus einem Metall mit einer niedrigeren Vickers-Härte bestehen als die Schraubverbindung.

Hierdurch kann eine Verzahnung durch Verformung der Abstandhalter erfolgen und eine variable Beabstandung der Module erreichet werden, wenn eine Vielzahl unterschiedlich hoher Abstandhalter vorgesehen ist. Die Verzahnung mit Verformung hat den Vorteil, dass sie besonders stabil ist, hat aber den Nachteil, dass bei einer plastischen Verformung stets die Gefahr besteht, dass sich Teile ablösen.

Gemäß eine bevorzugten Ausführung der Erfindung kann vorgesehen sein, dass das Gelenkspacer-System ein Hüftspacer-System ist und drei Module aufweist, wobei das dritte Modul ein Adaptermodul ist, das im zusammengesetzten Zustand das erste Modul vom zweiten Modul beabstandet und aneinander unlösbar befestigt.

Dabei kann vorgesehen sein, dass das Adaptermodul gewinkelt ist und einen Winkel zwischen 110° und 145° aufweist.

Des Weiteren kann bei allen erfindungsgemäßen Gelenkspacer-Systemen bevorzugt vorgesehen sein, dass die im zusammengesetzten Zustand äußeren Oberflächen der Module durch einen Kunststoff gebildet sind, insbesondere durch einen Polymethylmethacrylat-Knochenzement gebildet sind, wobei in dem Kunststoff zumindest ein Antiinfektivum und/oder Antiseptikum suspendiert und/oder gelöst ist oder sind und wobei die Gewinde der Schraubverbindungen und der Schraubensicherungen nicht durch den Kunststoff gebildet sind, sondern bevorzugt aus Metall bestehen.

Hierdurch wird eine medizinische Wirkung der damit aufgebauten Gelenkspacer ermöglicht, ohne dass deren Stabilität reduziert würde. Antibiotika sind als Antiinfektiva besonders bevorzugt.

Gemäß einer bevorzugten Ausführungsform kann vorgesehen sein, dass das Gelenkspacer-System aus einem biokompatiblen Material besteht, die besonders bevorzugt eine aus ausgehärtetem Polymethylmethacrylat-Knochenzement bestehende Hülle aufweist, der mindestens ein Antibiotikum und/oder ein Antiseptikum enthält.

Diese Materialien sind besonders gut zur Verwendung im menschlichen Körper geeignet.

Mit einer Weiterbildung der Erfindung wird auch vorgeschlagen, dass das Gelenkspacer-System eine Vielzahl von unterschiedlichen Modulen gleicher Funktion aber unterschiedlicher äußerer Form aufweist, so dass aus dem Gelenkspacer-System eine Vielzahl unterschiedlicher Gelenkspacer zusammenbaubar ist.

Die unterschiedliche Form ist dabei nur an den nach außen gerichteten Abmessungen unterschiedlich. Die Schraubverbindungen und die Schraubensicherungen gleichartiger Module sind identisch, so dass die verschiedenen Module immer noch zu den verschiedenen anderen Modulen passen.

Die Aufgaben der Erfindung werden auch gelöst durch ein Verfahren zum Aufbau eines Gelenkspacers mit einem solchen Gelenkspacer-System, bei dem der Gelenkspacer aus wenigstens zwei Modulen zusammengeschraubt wird, die in Abhängigkeit von der Anatomie und/oder der Behandlungssituation aus einer Vielzahl von unterschiedlichen Modulen ausgewählt werden, wobei die Module derart miteinander verschraubt werden, dass die Schraubensicherungen ein Lösen oder Auseinanderschrauben der Module verhindert.

Schließlich werden die Aufgaben der Erfindung auch gelöst durch die Verwendung eines Gelenkspacers hergestellt aus einem solchen Gelenkspacer-System als temporärer Platzhalter für ein künstliches Gelenk.

Der Erfindung liegt die überraschende Erkenntnis zugrunde, dass es mit den Schraubensicherungen gelingt, die Module eines modularen Gelenkspacer-Systems über Schraubverbindungen ohne Aufwand fest und für den verwendeten Zweck endgültig miteinander zu verbinden, ohne dass hierzu eine Fixierung mit Knochenzement notwendig wäre. Besonders vorteilhaft sind dabei Zahnscheiben oder Keilsicherungen als Schraubensicherungen einsetzbar, die bevorzugt einteilig mit den Modulen des Gelenkspacer-Systems ausgebildet sind. Mit der Erfindung ist auch ein Verkleben der Module unnötig. Klebstoffe wirken sich häufig nachteilig auf den Heilungsprozess aus und sind daher nachteilig.

Die Erfindung kann beispielsweise ausgeführt werden durch ein Gelenkspacer-System aus Modulen, wobei jedes Modul bevorzugt mindestens einen Metallkern besitzt und diese Metallkerne am Ende als Elemente von Schraubverbindungen ausgebildet sind, an deren Ende die Metallkerne als Zahnscheiben oder Keilsicherungsteile ausgeformt sind. Das bedeutet, die Module können miteinander über die Elemente der Schraubverbindungen miteinander verschraubt werden, bis sich die angrenzenden Zahnscheiben oder Keile ineinander drehen. Durch Überdrehen der Flanken, die entgegen der Drehrichtung der Schraubverbindung angeordnet sind, ist ein Rückdrehen der Module nicht mehr möglich. Dadurch sind die Module irreversibel und verdrehsicher miteinander verbunden.

Ein erfindungsgemäßes, artikulierendes Gelenkspacer-System besteht beispielsweise aus mindestens zwei Modulen, wobei
a) jedes Modul mindestens ein Element der Schraubverbindungen enthält,
b) jedes Modul am Ende des mindestens einen Elements einer Schraubverbindung als Zahnscheibe oder Keilsicherungsteil ausgebildet ist, wobei die Flanken der Zähne der Zahnscheiben oder Keile Keilsicherungsteile entgegen der Drehrichtung des mindestens einen Elements der Schraubverbindungen angeordnet sind,
c) die Höhe der Zähne der Zahnscheiben oder Keile Keilsicherungsteile größer ist als die Ganghöhe des Gewindes der Schraubverbindungen und
d) das mindestens eine Element der Schraubverbindungen und die Zahnscheiben oder Keilsicherungsteile aus einem biokompatiblen Metall bestehen.

Es kann erfindungsgemäß vorgesehen sein, dass die Schraubverbindungen und die Zahnscheiben oder Keilsicherungsteile jedes Moduls stoffschlüssig miteinander verbunden sind, wobei bevorzugt die Schraubverbindungen und die Zahnscheiben oder Keilsicherungsteile einteilig, also einstückig ausgebildet sind.

Die Module enthalten bevorzugt mindestens jeweils einen Metallkern, wobei mindestens ein Ende des Metallkerns als Außengewinde oder Innengewinde ausgebildet ist, wobei der Metallkern an einem Ende des wenigstens einen Außengewindes oder Innengewindes als Zahnscheibe oder Keilsicherungsteil ausgeformt ist.

Erfindungsgemäß kann ein erfindungsgemäßes Hüftspacer-System zusammengesetzt sein aus
a) einem Kopfmodul mit Gleitfläche,
b) einem Schaftmodul und
c) mindestens einem Adaptermodul, wobei dieser das Kopfmodul vom Schaftmodul beabstandet.

In einer weiteren erfindungsgemäßen Ausführungsform ist das Hüftspacer-System zusammengesetzt aus
a) einem Kopfmodul mit Gleitfläche,
b) einem Schaftmodul und
c) einem gekrümmten Adaptermodul, das einen CCD-Winkel von 110°-145° hat.

Durch Verwendung von Adaptermodulen mit unterschiedlichen CCD-Winkeln können Patienten versorgt werden, deren CCD-Winkel deutlich von dem in Europa sehr häufig anzutreffenden CCD-Winkel von 135° abweichen.

Es kann weiterhin erfindungsgemäß vorgesehen sein, dass als Abstandhalter zwischen den Modulen Zahnscheiben oder Keilsicherungsscheiben angeordnet sind, die auf beiden Seiten eine Zahnung besitzen, wobei bevorzugt diese Abstandhalter aus einem Metall bestehen, das eine niedrigere Vickers-Härte besitzt als der Metallkern der Module. Diese zusätzlichen Abstandhalter sind nicht Teil der Module. Theoretisch können auch einfache Unterlegscheiben aus einen weicheren Material als Abstandhalter verwendet werden.

Es kann ferner erfindungsgemäß vorgesehen sein, dass der Metallkern ganz oder teilweise mit einem Kunststoff überzogen ist, in dem ein oder mehrere Antiinfektiva und/oder Antiseptika suspendiert und/oder gelöst sind, wobei die Außengewinde und/oder Innengewinde nicht mit einem Kunststoff überzogen sind. Als Antiinfektiva kommen alle üblichen Antibiotika in Betracht, wobei besonders Gentamicin, Tobramycin, Clindamycin, Vancomycin, Daptomycin und Fosfomycin erfindungsgemäß bevorzugt sind. Als Antiseptika sind besonders Polyhexanid, Octenidin, Chlorhexidin und Wasserstoffperoxid-abspaltende Salze oder Addukte bevorzugt.

Im Folgenden werden Ausführungsbeispiele der Erfindung anhand von sechs schematisch dargestellten Figuren erläutert, ohne jedoch dabei die Erfindung zu beschränken. Dabei zeigt:
Figur 1: eine schematische Querschnittansicht der Module eines erfindungsgemäßen zweiteiligen Hüftgelenkspacer-Systems;
Figur 2: eine schematische Aufsicht in Blickrichtung A auf die Verbindungsseite des Gelenkkopf-Moduls nach Figur 1;
Figur 3: eine schematische Aufsicht in Blickrichtung B auf die korrespondierende Verbindungsseite des Schaft-Moduls nach Figur 1;
Figur 4: eine schematische Querschnittansicht der Module eines erfindungsgemäßen dreiteiligen Hüftgelenkspacer-Systems;
Figur 5: eine schematische Seitenansicht eines Ausschnitts der Verbindungsstelle zweier verbundener Module eines erfindungsgemäßen Gelenkspacer-Systems; und
Figur 6: eine schematische Seitenansicht eines Ausschnitts der Verbindungsstelle zweier verbundener Module eines weiteren erfindungsgemäßen Gelenkspacer-Systems.

In den Figuren werden teilweise für gleiche oder gleichartige Bauteile die gleichen Bezugszeichen verwendet.

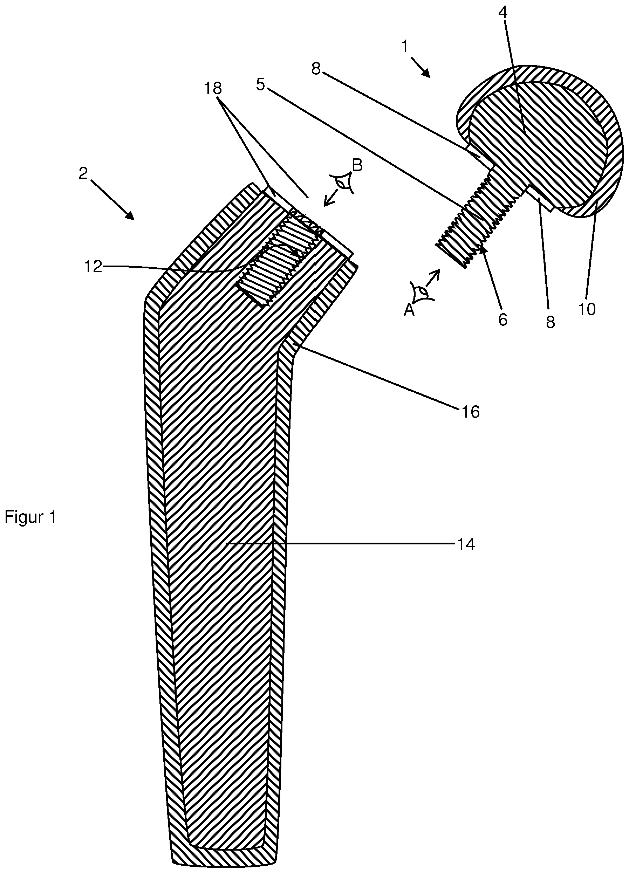

Figur 1 zeigt eine schematische Querschnittansicht der Module eines erfindungsgemäßen zweiteiligen Hüftgelenkspacer-Systems. Das Hüftgelenkspacer-System besteht aus einem Gelenkkopfmodul 1 und einem Schaftmodul 2, die miteinander verschraubbar sind. Das Gelenkkopfmodul 1 besteht in seinem Inneren aus einem Metallkern 4, der sich auf der Unterseite (weist in Figur 1 nach unten links), die mit dem Schaftmodul 2 verbunden werden soll, als Gewindestange 5 fortsetzt. Die Gewindestange 5 hat ein Außengewinde 6. Der Metallkern 4 besteht aus einem medizinischen Stahl, wie beispielsweise einem Chrom-Kobalt-Stahl.

An der Unterseite des Gelenkkopfmoduls 1 befindet sich als Abschluss der Gewindestange 5 ein Teil einer Keilsicherung 8, bei der mehrere Keile 8 umlaufend um die Verbindung der Gewindestange 5 zum Metallkern 4 am Metallkern 4 angeordnet sind. Bevorzugt sind die Keile 8 einteilig beziehungsweise einstückig mit dem Metallkern 4 ausgeführt. Alternativ kann die Keilsicherung 8 aber auch an dieser Stelle aufgeklebt oder aufgelötet sein. Die Keile 8 der Keilsicherung 8 weisen eine flache Seite und eine senkrechte Keilfläche auf.

Die Außenseite des Gelenkkopfmoduls 1, die als Gleitfläche des Hüftgelenkspacers dienen soll, wird durch eine Kunststoffschicht 10 aus einem Polymethylmethacrylat-Knochenzement (PMMA-Knochenzement) vollflächig abgedeckt. In dem PMMA-Knochenzement ist eine Mischung von zwei Antibiotika als Antiinfektivum enthalten, die aus dem PMMA-Knochenzement herauslösbar ist und zur Behandlung einer Infektion dient. Zudem wird durch die Verwendung der Kunststoffschicht 10 vermieden, dass sich Metallteile abreiben und Schwierigkeiten bei der Behandlung verursachen.

Die Gleitfläche wird im eingesetzten Zustand an einer gegebenenfalls ebenfalls künstlichen Hüftpfanne anliegen und dort die Funktion des Hüftgelenks übernehmen.

Das Schaftmodul 2 ist zur Verbindung mit einem Oberschenkelknochen des Patienten vorgesehen. Das Schaftmodul 2 weist eine zur Gewindestange 5 des Gelenkkopfmoduls 1 passende Bohrung oder ein passendes zylindrisches Loch auf, in dem ein zum Außengewinde 6 passendes Innengewinde 12 vorgesehen ist. Das Loch mit dem Innengewinde 12 ist in einem Metallkern 14 des Schaftmoduls 2 enthalten. Das Innengewinde 12 und das Außengewinde 6 bilden mit dem Loch und der Gewindestange 5 eine Schraubverbindung, mit der das Gelenkkopfmodul 1 mit dem Schaftmodul 2 verbindbar sind. Im miteinander verbundenen Zustand bilden die beiden Module 1, 2 des Hüftgelenkspacer-Systems einen Hüftgelenkspacer zum temporären Ersetzen eines künstlichen Hüftgelenks.

Bis auf die Verbindungsfläche des Schaftmoduls 2 zum Gelenkkopfmodul 1 (weist in Figur 1 nach oben rechts), ist die Oberfläche des Schaftmoduls 2 vollständig mit einer Kunststoffschicht 16 aus einem PMMA-Knochenzement abgedeckt. An der Verbindungsfläche des Schaftmoduls 2, von der aus sich auch das Loch in den Metallkern 14 erstreckt, liegt der Metallkern 14 offen. Auf der Oberfläche des Metallkerns 14 der Verbindungsfläche sind die Keile 18 des zweiten Teils der Keilsicherung 18 vorgesehen, die mit den Keilen 8 des Gelenkkopfmoduls 1 eine gemeinsame Keilsicherung 18 bilden. Dazu sind die Keile 18 um die Öffnung zu dem Loch herum angeordnet. Die beiden Teile der Keilsicherung 8, 18 greifen ineinander, wenn das Gelenkkopfmodul 1 vollständig in das Schaftmodul 2 geschraubt ist und verhindern über die senkrechten Keilflächen der Keile 8, 18 ein Lösen der Verbindung.

Bevorzugt sind die Keile 18 einteilig beziehungsweise einstückig mit dem Metallkern 14 ausgeführt. Alternativ kann die Keilsicherung 18 aber auch an dieser Stelle aufgeklebt oder aufgelötet sein. Die Keile 18 der Keilsicherung 18 weisen eine flache Seite und eine senkrechte Keilfläche auf, wobei die Steigungen und die Höhen der flachen Keilflächen und der senkrechten Keilflächen die Gleichen, wie die der Keile 8 des Gelenkkopfmoduls 1 sind. Grundsätzlich bilden die Keile 8, 18 ein zueinander passendes Keilsicherungsscheibenpaar, wobei die Keilsicherungsscheiben fest mit den Metallkernen 4, 14 der beiden Module 1, 2 an der Basis von deren Gewinden 6, 12 der Schraubverbindung verbunden sind oder bevorzugt sogar einteilig mit den Metallkernen 4, 14 der beiden Module 1, 2 an der Basis von deren Gewinden 6, 12 der Schraubverbindung ausgeführt sind.

Das Schaftmodul 2 ist mit einem CCD-Winkel von 142° gewinkelt und damit der Form eines speziellen und nicht für Europa typischen Oberschenkelknochens nachempfunden. Alternativ dazu kann auch ein anderes Schaftmodul (nicht gezeigt) mit einem anderen CCD-Winkel, beispielsweise mit dem europäischen Normalwinkel von 135° ausgewählt werden. Ebenso kann statt dem Gelenkkopfmodul 1 ein anderes Gelenkkopfmodul (nicht gezeigt) mit einer anderen Form und/oder Größe ausgewählt werden. Zur Behandlung liegen eine Vielzahl verschiedener Schaftmodule und Gelenkkopfmodule als Set vor, die jedoch immer die gleichen Gewindebohrungen und Gewindestangen 5 aufweisen und daher immer miteinander kompatibel, das heißt, miteinander verschraubbar sind. Durch diese Maßnahme können unterschiedliche Formen von Hüftgelenkspacern mit dem Hüftgelenkspacer-System erzeugt werden. Durch die durch die Keilsicherung 8, 18 gebildete Schraubensicherung 8, 18 wird dabei sichergestellt, dass sich die beiden Module 1, 2 nicht mehr voneinander lösen lassen, ohne dass hierzu eine Zementierung der beiden Teile erfolgen müsste.

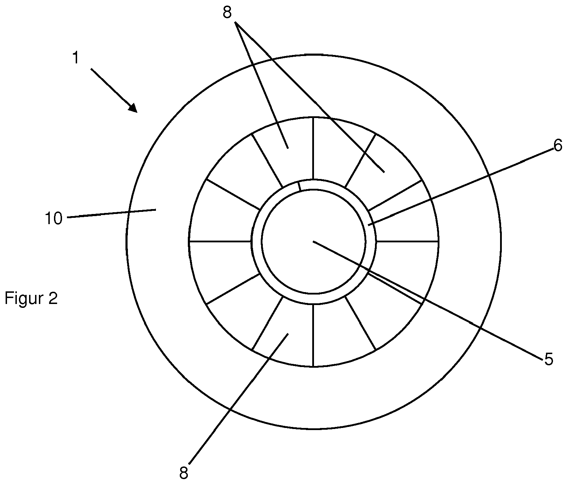

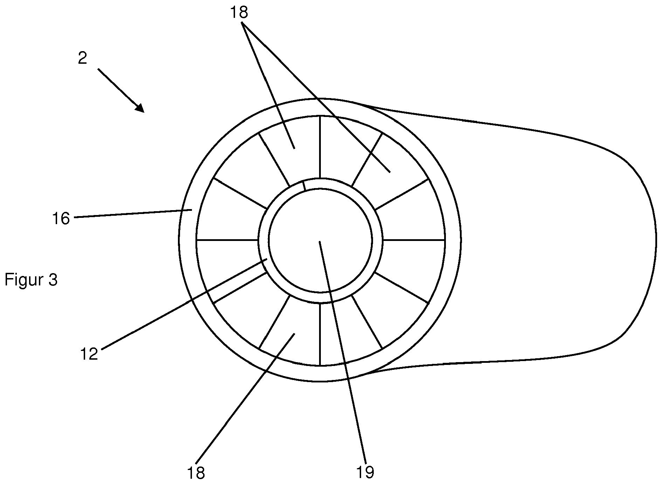

Figur 2 zeigt eine schematische Aufsicht in Blickrichtung A auf die Verbindungsseite des Gelenkkopfmoduls 1 nach Figur 1 und Figur 3 zeigt eine schematische Aufsicht in Blickrichtung B auf die korrespondierende Verbindungsseite des Schaftmoduls 2 nach Figur 1. Die Blickrichtungen A und B sind in Figur 1 mit einem schematisch dargestellten Auge gekennzeichnet, um die Blickrichtung zu verdeutlichen.

In Figur 2 weist die Gewindestange 5 auf den Betrachter zu (aus der Bildebene hinaus). Außen um die Gewindestange 5 ist das Außengewinde 6 zu erkennen. Analog dazu erstreckt sich das Loch 19 beziehungsweise die Gewindebohrung 19 senkrecht von der Bildebene in die Bildebene hinein, das heißt vom Betrachter weg. Am Rand des Lochs 19 ist das Innengewinde 12 zu erkennen.

Die Keile 8, 18 sind konzentrisch und bezogen auf die Symmetrieachse der Gewindestange 5 und des Gewindebohrung 19 mit einer zwölfzähligen Drehsymmetrieachse um die Gewindestange 5 beziehungsweise die Gewindebohrung 19 herum angeordnet. Die senkrechten Linien zwischen den Keilen 8, 18 der Keilsicherungsteile 8, 18 entsprechen den senkrechten Keilflächen der Keile 8, 18. Während die Flächen einer Sicht auf die flachen Keilflächen entsprechen.

Der Fortsatz an dem Schaftmodul 2, der sich in Figur 3 nach rechts erstreckt, ist der abgewinkelte Schaft des Schaftmoduls 2. Durch ein Set mit Modulen 1, 2 mit verschiedenen äußeren Formen und Abmessungen des Schaftmoduls 2 und des Gelenkkopfmoduls 1 können auf einfache Weise Hüftgelenkspacer hergestellt werden, die an die erforderliche Behandlungssituation angepasst sind. Durch die erfindungsgemäßen Schraubensicherungen 8, 18 können derartig zusammengeschraubte Hüftgelenkspacer direkt beim Patienten eingesetzt werden, ohne dass eine zusätzliche Fixierung der beiden Module 1, 2 erfolgen müsste.

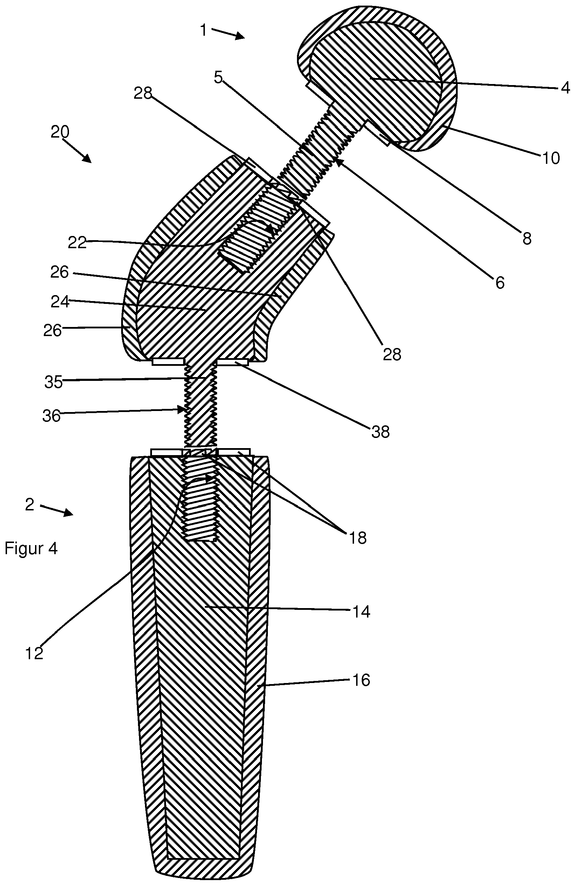

Figur 4 zeigt eine schematische Querschnittansicht der Module eines erfindungsgemäßen dreiteiligen Hüftgelenkspacer-Systems. Das Hüftgelenkspacer-System nach Figur 4 unterscheidet sich von dem nach den Figuren 1 bis 3 dadurch, dass zusätzlich zu einem Gelenkkopfmodul 1 und einem Schaftmodul 2 noch ein drittes Modul 20, nämlich ein Adaptermodul 20 vorgesehen ist.

Das Gelenkkopfmodul 1 ist analog dem Gelenkkopfmodul nach Figur 1 und 2 aufgebaut. Als Stahl für den Metallkern 4, die Gewindestange 5 und die Keile 8 wird hier ein V4A-Stahl verwendet. Die Gleitfläche und die Außenseite werden bis auf die Verbindungsfläche zum Adaptermodul 20 mit PMMA-Knochenzement enthaltend wenigstens eine pharmazeutisch aktive Substanz abgedeckt.

Das Schaftmodul 2 weist im Unterschied zu dem nach den Figuren 1 und 3 keinen CCD-Winkel auf, da dieser vorliegend mit Hilfe des Adaptermoduls 20 vorgegeben wird. Das Schaftmodul 2 nach Figur 4 weist einen Metallkern 14 und Keile 18 einer Keilsicherung aus einem V4A-Stahl auf, der auf der Außenseite bis auf die Verbindungsfläche zum Adaptermodul (in Figur 4 oben) mit einem PMMA-Knochenzement abgedeckt ist. In dem Metallkern 14 ist eine Gewindebohrung mit einem Innengewinde 12 vorgesehen.

Das Adaptermodul 20 hat eine Gewindebohrung mit einem Innengewinde 22 zur Verbindung mit dem Gelenkkopfmodul 1. Die Gewindebohrung ist in einem Metallkern 24 aus einem V4A-Stahl (beispielsweise 1.4401, 1.4404 oder 1.4571) vorgesehen. Die Metallkerne 4, 14, 24 bestehen aus dem gleichen V4A-Stahl.

Die Außenseite des Adaptermoduls 20 ist bis auf die Verbindungsfläche zum Gelenkkopfmodul 1 (in Figur 4 nach oben rechts) und die Verbindungsfläche zum Schaftmodul 2 (in Figur 4 nach unten) mit einem PMMA-Knochenzement 26 umhüllt. In den PMMA-Knochenzementhüllen 10, 16, 26 ist wenigstens ein Antibiotikum und/oder wenigstens ein Antiseptikum enthalten, das aus den PMMA-Knochenzementhüllen 10, 16, 26 zur Behandlung des umliegenden menschlichen Gewebes im beim Patienten eingesetzten Zustand über einen Zeitraum von mehreren Tagen oder Wochen herauslösbar ist.

Auf der Verbindungsfläche zum Gelenkkopfmodul 1 sind konzentrisch und drehsymmetrisch um die Gewindebohrung herum Keile 28 angeordnet, die zu den Keilen 8 des Gelenkkopfmoduls 1 passen und die mit den Keilen 8 eine Keilsicherung bilden. Auf der Verbindungsfläche zum Schaftmodul 2 ist eine Gewindestange 15 mit einem Außengewinde 36 vorgesehen, das zu dem Innengewinde 12 des Schaftmoduls 2 passt. Ebenso sind auf der Verbindungsfläche zum Schaftmodul 2 Keile 38 vorgesehen, die zu den Keilen 18 des Schaftmoduls 2 passen und mit diesen eine zweite Keilsicherung bilden.

Wenn die Module 1, 2, 20 ineinander geschraubt werden, greifen die Keile 8, 28 und die Keile 18, 38 ineinander und verbinden sich miteinander. Durch den Formschluss der Keile 8, 28 und der Keile 18, 38 können die Module 1, 2, 20 nicht mehr auseinander geschraubt werden. Die senkrechten Seiten (die Flanken) der Keile 8, 28 und der Keile 18, 38 stehen der Drehbewegung beim Lösen der Schraubverbindung entgegen, während die flachen Seiten der Keile 8, 28 und der Keile 18, 38 beim Zusammenschrauben der Schraubverbindungen 8, 18, 28, 38 noch übereinander gleiten können, so dass die Verzahnung erst beim Lösen der Schrauben greift.

Im Inneren der Gewindebohrungen nach den Figuren 1 und 4 sind die Seitenansichten der Innengewinde 12, 22 dargestellt, das heißt, dass die Strukturen im Inneren der Gewindebohrungen keine Schraffur zur Darstellung eines geschnittenen Materials darstellen sondern eben die Rillen der Innengewinde 12, 22. Bei den Querschnittansichten sind die geschnittenen Flächen der Gelenkspacer-Systeme beziehungsweise der Module 1, 2, 20 immer schraffiert dargestellt.

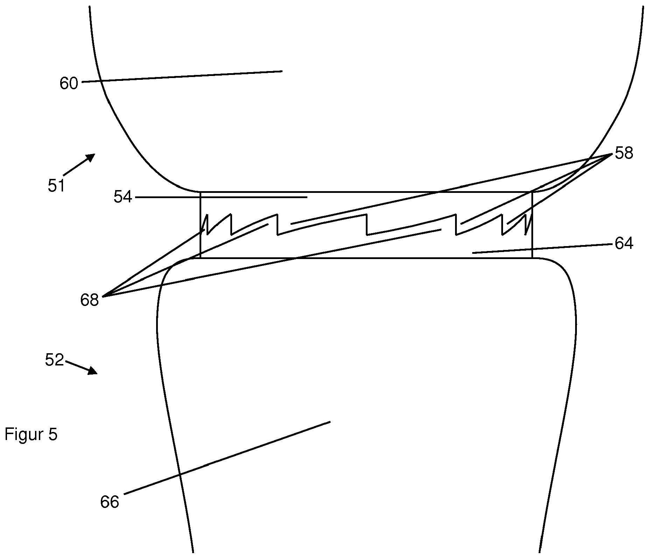

Figur 5 zeigt eine schematische Seitenansicht eines Ausschnitts der Verbindungsstelle zweier verbundener Module 51, 52 eines erfindungsgemäßen Gelenkspacer-Systems. Die Module 51, 52 weisen an den zueinander weisenden Flächen jeweils ein Teil eines Keilsicherungsscheibenpaars 54, 64 auf, die an den Modulen 51, 52 befestigt sind. Jede Keilsicherungsscheibe 54, 64 weist an den zueinander weisenden Flächen Keile 58, 68 auf, die im zusammengesetzten Zustand ineinander greifen.

Eine der Keilsicherungsscheiben 54, 64 kann drehbar an einem der Module 51, 52 gelagert sein, wobei bei ausreichendem Druck durch das Zusammenschrauben die Keilsicherungsscheibe 54, 64 entweder arretiert oder durch den Anpressdruck nach erfolgtem Zusammenschrauben sich mit dem Modul 51, 52 verpresst. Hierdurch kann sichergestellt werden, dass die Keilsicherungsscheiben 54, 64 leicht ineinander greifen können. Eine Drehbarkeit einer der Keilsicherungsscheiben 54, 64 muss aber nicht gegeben sein, da die elastische Verformung der Keile 58, 68 ausreicht, damit die Keile 58, 68 derart ineinander greifen, dass sie in der Gegendrehrichtung nicht mehr lösbar sind.

Die steilen (senkrechten) Flanken der Keile 58, 68 sind entgegen der Drehrichtung der Schraubverbindung der beiden Module 51, 52 ausgerichtet und die Ganghöhe des Gewindes der Schraubverbindung ist kleiner als die Höhe der Keile 58, 68 der Keilsicherungsscheiben 54, 64. Die Module sind außen mit einem PMMA-Knochenzement 60, 66 verkleidet, in dem lösbare Antibiotika enthalten sind.

Alternativ zu den in den Ausführungsbeispielen nach den Figuren 1 bis 5 gezeigten Keilsicherungen können auch andere Schraubensicherungen verwendet werden, um ein Lösen ineinander geschraubter Module zu verhindern. Beispielsweise können Sperrzähne aus einem harten Metall vorgesehen sein, die sich in einen weicheren Bereich im Bereich der Basis der Gewindebohrung oder der Gewindestange eingraben und ein Rückdrehen verhindern. Ebenso kann eine federnde Schnorrscheibe einseitig an der Basis der Gewindestange mit dem Metallkern verbunden sein (beispielsweise angeschweißt sein). Die Zähne (beziehungsweise Keile) der federnden Schnorrscheibe können entweder in eine federnde Schnorrscheibe an dem anderen, zu verbindenden Modul greifen und sich damit verbinden oder die Zähne greifen in ein weicheres Material des gegenüberliegenden Metallkerns. Durch die Federeigenschaften sind die Module einfacher und mit weniger Kraftaufwand miteinander verbindbar.

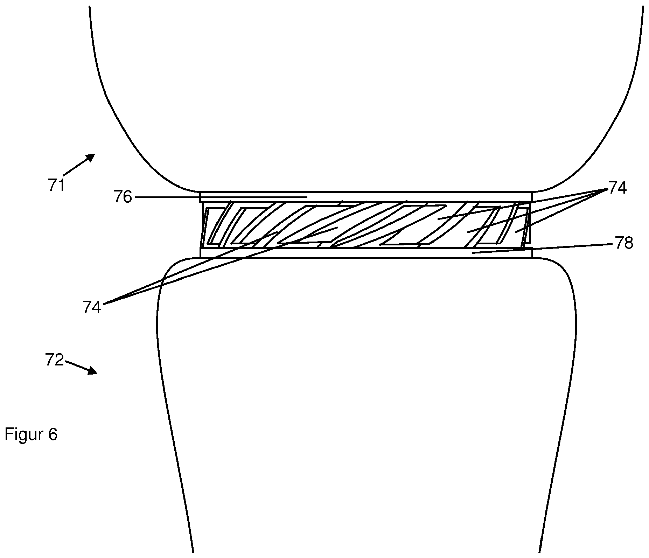

Figur 6 zeigt eine zeigt eine schematische Seitenansicht eines Ausschnitts der Verbindungsstelle zweier verbundener Module 71, 72 eines erfindungsgemäßen Gelenkspacer-Systems, bei dem als Schraubensicherungen schrägstehende elastische Metallscheiben 74 nach der Art von Zahnscheiben mit den Modulen 71, 72 verbunden sind. Die Metallscheiben 74 entsprechen den Keilen 8, 18, 28, 38, 58, 68 nach den Figuren 1 bis 5 und erfüllen deren Funktion, wobei hier die senkrechten Flanken fehlen und die schrägstehenden Flanken durch eine Seite der Metallscheiben 74 gebildet werden.

Die Metallscheiben 74 sind die Zähne 74 der Zahnscheiben. Dadurch können sich die Metallscheiben 74 beim Zusammenschrauben (oberes Modul 71 wird nach rechts, das heißt, gegen den Uhrzeigersinn gegen das untere Modul 72 gedreht) elastisch verformen, während die Metallscheiben 74 beim Auseinanderschrauben (oberes Modul 71 wird nach links, das heißt, mit dem Uhrzeigersinn gegen das untere Modul 72 gedreht) der Module 71, 72 in ineinandergreifen und ein Lösen der Module 71, 72 verhindern.

Die Metallscheiben 74 sind rotations-drehsymmetrisch zu einer zentralen Verschraubung (Gewindestange und Gewindebohrung, in Figur 6 nicht zu sehen) angeordnet. Die Metallscheiben 74 sind in den gezeigten Neigungswinkeln entlang ihrer Oberkanten an einem Metallkern 76 des oberen Moduls 71 beziehungsweise an ihren Unterkanten an einem Metallkern 78 des unteren Moduls 72 angeschweißt oder bevorzugt einteilig mit den jeweiligen Metallkernen 76, 78 aufgebaut.

Die Metallscheiben 74 lassen sich aufgrund ihrer Elastizität ineinander schrauben. Bei dem Versuch, die Module 71, 72 durch eine gegensinnige Drehung wieder zu lösen, greifen die Metallscheiben 74 aber ineinander und blockieren die Drehung. Die Module 71, 72 sind dann unlösbar miteinander verbunden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 51, 71: Gelenkkopfmodul / Erstes Modul
- 2, 52, 72: Schaftmodul / Zweites Modul
- 4, 14, 24: Metallkern
- 5, 35: Gewindestange
- 6, 36: Außengewinde
- 8, 18, 28, 38: Keil / Keilsicherung
- 10, 16, 26: Kunststoffschicht
- 12, 22: Innengewinde
- 19: Loch / Gewindebohrung
- 20: Adaptermodul / Drittes Modul
- 54, 64: Metallkern
- 58, 68: Verzahnung
- 60, 66: PMMA-Hülle
- 74: Metallscheibe
- 76, 78: Metallkern
- A: Blickrichtung der Aufsicht nach Figur 2
- B: Blickrichtung der Aufsicht nach Figur 3

## Patentansprüche

1. Modulares Gelenkspacer-System zum Zusammenschrauben eines Gelenkspacers, der zum zeitweisen Ersetzen eines künstlichen Gelenks vorgesehen ist, wobei das Gelenkspacer-System zumindest zwei über eine Schraubverbindung (5, 6, 12, 22, 35, 36) verbindbare Module (1, 2, 20, 51, 52, 71, 72) aufweist, wobei ein erstes Modul (1, 51, 71) des Gelenkspacer-Systems eine Oberfläche zur Ausbildung einer Gleitfläche des Gelenks des Gelenkspacers aufweist und ein zweites Modul (2, 52, 72) des Gelenkspacer-Systems einen Schaft zur Verbindung mit einem Knochen aufweist, wobei die über die Schraubverbindung (5, 6, 12, 22, 35, 36) verbindbaren Module (1, 2, 20, 51, 52, 71, 72) für jede Schraubverbindung (5, 6, 12, 22, 35, 36) zumindest eine Schraubensicherung (8, 18, 28, 38, 58, 68, 74) aufweisen, wobei die zumindest eine Schraubensicherung (8, 18, 28, 38, 58, 68, 74) mit einem der Module (1, 2, 20, 51, 52, 71, 72) verbunden ist oder einteilig mit einem Modul (1, 2, 20, 51, 52, 71, 72) ausgeführt ist, und wobei zu jeder Schraubverbindung (5, 6, 12, 22, 35, 36) zwei Schraubensicherungsteile (8, 18, 28, 38, 58, 68, 74) vorgesehen sind, **dadurch gekennzeichnet, dass** die zwei Schraubensicherungsteile fest mit den beiden über die Schraubverbindung (5, 6, 12, 22, 35, 36) zu verbindenden Modulen (1, 2, 20, 51, 52, 71, 72) verbunden sind oder einteilig mit den beiden über die Schraubverbindung (5, 6, 12, 22, 35, 36) zu verbindenden Modulen (1, 2, 20, 51, 52, 71, 72) ausgeführt sind und dass die zwei Schraubensicherungsteile beim Zusammenschrauben der Module (1, 2, 20, 51, 52, 71, 72) derart ineinander greifen, dass sie einem Lösen der Schraubverbindung (5, 6, 12, 22, 35, 36) entgegenwirken, bevorzugt ein Lösen der Schraubverbindung (5, 6, 12, 22, 35, 36) verhindern.

2. Gelenkspacer-System nach Anspruch 1, **dadurch gekennzeichnet, dass**
das Gelenkspacer-System zumindest drei über Schraubverbindungen (5, 6, 12, 22, 35, 36) verbindbare Module (1, 2, 20, 51, 52, 71, 72) aufweist und zumindest zwei Module (1, 2, 20, 51, 52, 71, 72) wenigstens eine Schraubensicherung (8, 18, 28, 38, 58, 68, 74) aufweisen.

3. Gelenkspacer-System nach einem der vorangehenden Ansprüche, **dadurch gekennzeichnet, dass**
jede Schraubverbindung (5, 6, 12, 22, 35, 36) ein Innengewinde (12, 22) und ein zum Innengewinde (12, 22) passendes Außengewinde (6, 36) aufweist, und zwei über eine Schraubverbindung (5, 6, 12, 22, 35, 36) miteinander verbindbare Module (1, 2, 20, 51, 52, 71, 72) an den Enden, die im miteinander verbundenen Zustand zueinander weisen, jeweils das Innengewinde (12, 22) und das Außengewinde (6, 36) dieser Schraubverbindung (5, 6, 12, 22, 35, 36) aufweisen, so dass die Schraubverbindung (5, 6, 12, 22, 35, 36) ein Teil der beiden Module (1, 2, 20, 51, 52, 71, 72) ist.

4. Gelenkspacer-System nach Anspruch 3, **dadurch gekennzeichnet, dass**
an jeweils einem der miteinander verbindbaren Module (1, 2, 20, 51, 52, 71, 72) am Ende des Außengewindes (6, 36) oder am Anfang des Innengewindes (12, 22) die Schraubensicherung (8, 18, 28, 38, 58, 68, 74) vorgesehen ist oder an beiden miteinander verbindbaren Modulen (1, 2, 20, 51, 52, 71, 72) am Ende des Außengewindes (6, 36) oder am Anfang des Innengewindes (12, 22) ein Schraubensicherungsteil (8, 18, 28, 38, 58, 68, 74) vorgesehen ist.

5. Gelenkspacer-System nach einem der Ansprüche 3 bis 4, **dadurch gekennzeichnet, dass**
die Module (1, 2, 20, 51, 52, 71, 72) jeweils einen Metallkern (4, 14, 24, 54, 64, 76, 78) aufweisen, wobei der Metallkern (4, 14, 24, 54, 64, 76, 78) aus einem der zueinander weisenden Enden der miteinander verbindbaren Module (1, 20, 51, 52, 71, 72) herausragt und dort das Außengewinde (6, 36) bildet und in dem Metallkern (4, 14, 24, 54, 64, 76, 78) des anderen Moduls (2, 20, 51, 52, 71, 72), der miteinander verbindbaren Module (1, 2, 20, 51, 52, 71, 72) ein Loch (19) mit dem Innengewinde (12, 22) vorgesehen ist, so dass das Außengewinde (6, 36) und das Innengewinde (12, 22) eine Schraubverbindung (5, 6, 12, 22, 35, 36) bilden.

6. Gelenkspacer-System nach einem der vorangehenden Ansprüche, **dadurch gekennzeichnet, dass**
auch die Schraubensicherung (8, 18, 28, 38, 58, 68, 74) durch wenigstens einen der Metallkerne (4, 14, 24, 54, 64, 76, 78) gebildet ist, bevorzugt die Schraubensicherung (8, 18, 28, 38, 58, 68, 74) durch die beiden Metallkerne (4, 14, 24, 54, 64, 76, 78) gebildet ist.

7. Gelenkspacer-System nach einem der vorangehenden Ansprüche, **dadurch gekennzeichnet, dass**
die zumindest eine Schraubensicherung (8, 18, 28, 38, 58, 68, 74) zwei Zahnscheiben (74) oder eine zweiteilige Keilsicherung (8, 18, 28, 38, 58, 68) ist.

8. Gelenkspacer-System nach Anspruch 7, **dadurch gekennzeichnet, dass**
die Flanken der Zähne (74) der Zahnscheiben (74) oder der Keile (8, 18, 28, 38, 58, 68) der Keilsicherungen (8, 18, 28, 38, 58, 68) entgegen der Drehrichtung der Elemente der zumindest einen Schraubverbindung (5, 6, 12, 22, 35, 36) geneigt sind und die Höhe der Zähne (74) der Zahnscheiben (74) oder der Keile (8, 18, 28, 38, 58, 68) der Keilsicherungen (8, 18, 28, 38, 58, 68) größer ist, als die Ganghöhe der Gewinde (5, 6, 35, 36) der Elemente der zumindest einen Schraubverbindung (5, 6, 12, 22, 35, 36).

9. Gelenkspacer-System nach einem der vorangehenden Ansprüche, **dadurch gekennzeichnet, dass**
jede Schraubverbindung (5, 6, 12, 22, 35, 36) und jede Schraubensicherung (8, 18, 28, 38, 58, 68, 74) aus einem biokompatiblen Material besteht.

10. Gelenkspacer-System nach einem der vorangehenden Ansprüche, **dadurch gekennzeichnet, dass**
zwischen den Modulen (1, 2, 20, 51, 52, 71, 72) Abstandhalter in Form von Zahnscheiben mit beidseitiger Zahnung oder Keilsicherungen mit beidseitigen Keilen vorgesehen sind, wobei bevorzugt die Abstandhalter aus einem Metall mit einer niedrigeren Vickers-Härte bestehen als die Schraubverbindung (5, 6, 12, 22, 35, 36).

11. Gelenkspacer-System nach einem der vorangehenden Ansprüche, **dadurch gekennzeichnet, dass**
das Gelenkspacer-System ein Hüftspacer-System ist und drei Module (1, 2, 20, 51, 52, 71, 72) aufweist, wobei das dritte Modul (20) ein Adaptermodul (20) ist, das im zusammengesetzten Zustand das erste Modul (1) vom zweiten Modul (2) beabstandet und aneinander unlösbar befestigt.

12. Gelenkspacer-System nach Anspruch 11, **dadurch gekennzeichnet, dass**
das Adaptermodul (20) gewinkelt ist und einen Winkel zwischen 110° und 145° aufweist.

13. Gelenkspacer-System nach einem der vorangehenden Ansprüche, **dadurch gekennzeichnet, dass**
die im zusammengesetzten Zustand äußeren Oberflächen der Module (1, 2, 20, 51, 52, 71, 72) durch einen Kunststoff (10, 16, 26, 60, 66) gebildet sind, insbesondere durch einen Polymethylmethacrylat-Knochenzement (10, 16, 26, 60, 66) gebildet sind, wobei in dem Kunststoff (10, 16, 26, 60, 66) zumindest ein Antiinfektivum und/oder Antiseptikum suspendiert und/oder gelöst ist oder sind und wobei die Gewinde (5, 6, 35, 36) der Schraubverbindungen (5, 6, 12, 22, 35, 36) und der Schraubensicherungen (8, 18, 28, 38, 58, 68, 74) nicht durch den Kunststoff (10, 16, 26, 60, 66) gebildet sind, sondern bevorzugt aus Metall bestehen.

14. Gelenkspacer-System nach einem der vorangehenden Ansprüche, **dadurch gekennzeichnet, dass**
das Gelenkspacer-System eine Vielzahl von unterschiedlichen Modulen (1, 2, 20, 51, 52, 71, 72) gleicher Funktion aber unterschiedlicher äußerer Form aufweist, so dass aus dem Gelenkspacer-System eine Vielzahl unterschiedlicher Gelenkspacer zusammenbaubar ist.

15. Verfahren zum Aufbau eines Gelenkspacers mit einem Gelenkspacer-System nach einem der vorangehenden Ansprüche, **dadurch gekennzeichnet, dass**
der Gelenkspacer aus wenigstens zwei Modulen (1, 2, 20, 51, 52, 71, 72) zusammengeschraubt wird, die in Abhängigkeit von der Anatomie und/oder der Behandlungssituation aus einer Vielzahl von unterschiedlichen Modulen (1, 2, 20, 51, 52, 71, 72) ausgewählt werden, wobei die Module (1, 2, 20, 51, 52, 71, 72) derart miteinander verschraubt werden, dass die Schraubensicherungen (8, 18, 28, 38, 58, 68, 74) ein Lösen oder Auseinanderschrauben der Module (1, 2, 20, 51, 52, 71, 72) verhindert.

## Claims

1. A modular articular spacer system for screwing together an articular spacer for replacing an artificial joint temporarily, wherein the articular spacer system comprises at least two modules (1, 2, 20, 51, 52, 71, 72) that are connectable by means of a screw connection (5, 6, 12, 22, 35, 36), wherein a first module (1, 51, 71) of the articular spacer system comprises a surface for formation of a sliding surface of the joint of the articular spacer and a second module (2, 52, 72) of the articular spacer system comprises a stem for connection to a bone, wherein the modules (1, 2, 20, 51, 52, 71, 72) connectable by means of the screw connection (5, 6, 12, 22, 35, 36) comprise at least one screw-locking device (8, 18, 28, 38, 58, 68, 74) for each screw connection (5, 6, 12, 22, 35, 36), wherein the at least one screw-locking device (8, 18, 28, 38, 58, 68, 74) is connected to one of the modules (1, 2, 20, 51, 52, 71, 72) or is provided in one part with a module (1, 2, 20, 51, 52, 71, 72), and wherein two screw-locking device parts (8, 18, 28, 38, 58, 68, 74) are provided for each screw connection (5, 6, 12, 22, 35, 36), **characterised in that** the two screw-locking device parts are fixedly connected to the two modules (1, 2, 20, 51, 52, 71, 72) to be connected by means of the screw connection (5, 6, 12, 22, 35, 36) or are formed in one part with the two modules (1, 2, 20, 51, 52, 71, 72) to be connected by means of the screw connection (5, 6, 12, 22, 35, 36), and **in that** the two screw-locking device parts engage in each other when the modules (1, 2, 20, 51, 52, 71, 72) are screwed together, in such a way that detachment of the screw connection (5, 6, 12, 22, 35, 36) is resisted, preferably detachment of the screw connection (5, 6, 12, 22, 35, 36) is prevented.

2. The articular spacer system according to claim 1, **characterised in that** the articular spacer system comprises at least three modules (1, 2, 20, 51, 52, 71, 72) that are connectable by means of screw connections (5, 6, 12, 22, 35, 36), and at least two modules (1, 2, 20, 51, 52, 71, 72) comprise at least one screw-locking device (8, 18, 28, 38, 58, 68, 74).

3. The articular spacer system according to any one of the preceding claims, **characterised in that** each screw connection (5, 6, 12, 22, 35, 36) comprises an internal thread (12, 22) and an external thread (6, 36) that matches the internal thread (12, 22), and two modules (1, 2, 20, 51, 52, 71, 72) connectable to each other by means of a screw connection (5, 6, 12, 22, 35, 36), on the ends facing each other in the connected state, comprise in one instance the internal thread (12, 22) and in the other instance the external thread (6, 36) of this screw connection (5, 6, 12, 22, 35, 36), such that the screw connection (5, 6, 12, 22, 35, 36) is a part of the two modules (1, 2, 20, 51, 52, 71, 72).

4. The articular spacer system according to claim 3, **characterised in that** the screw-locking device (8, 18, 28, 38, 58, 68, 74) is provided on one of the modules (1, 2, 20, 51, 52, 71, 72) connectable to each other, at the end of the external thread (6, 36) or at the start of the internal thread (12, 22) as appropriate, or a screw-locking device part (8, 18, 28, 38, 58, 68, 74) is provided on both modules (1, 2, 20, 51, 52, 71, 72) connectable to each other, at the end of the external thread (6, 36) or at the start of the internal thread (12, 22) .

5. The articular spacer system according to either one of claims 3 or 4, **characterised in that** each of the modules (1, 2, 20, 51, 52, 71, 72) comprises a metal core (4, 14, 24, 54, 64, 76, 78), wherein the metal core (4, 14, 24, 54, 64, 76, 78) projects from one of the ends, which face each other, of the modules (1, 20, 51, 52, 71, 72) connectable to each other and forms the external thread (6, 36) there, and in the metal core (4, 14, 24, 54, 64, 76, 78) of the other module (2, 20, 51, 52, 71, 72) of the modules (1, 2, 20, 51, 52, 71, 72) connectable to each other there is provided a hole (19) having the internal thread (12, 22), such that the external thread (6, 36) and the internal thread (12, 22) form a screw connection (5, 6, 12, 22, 35, 36).

6. The articular spacer system according to any one of the preceding claims, **characterised in that** the screw-locking device (8, 18, 28, 38, 58, 68, 74) is also formed by at least one of the metal cores (4, 14, 24, 54, 64, 76, 78), preferably the screw-locking device (8, 18, 28, 38, 58, 68, 74) is formed by the two metal cores (4, 14, 24, 54, 64, 76, 78) .

7. The articular spacer system according to any one of the preceding claims, **characterised in that** the at least one screw-locking device (8, 18, 28, 38, 58, 68, 74) is constituted by two tooth lock washers (74) or a two-part wedge lock (8, 18, 28, 38, 58, 68).

8. The articular spacer system according to claim 7, **characterised in that** the flanks of teeth (74) of the tooth lock washers (74) or the wedges (8, 18, 28, 38, 58, 68) of the wedge locks (8, 18, 28, 38, 58, 68) are inclined against a direction of rotation of the elements of the at least one screw connection (5, 6, 12, 22, 35, 36) and the height of the teeth (74) of the tooth lock washers (74) or of the wedges (8, 18, 28, 38, 58, 68) of the wedge locks (8, 18, 28, 38, 58, 68) is greater than the pitch of the threads (5, 6, 35, 36) of the elements of the at least one screw connection (5, 6, 12, 22, 35, 36).

9. The articular spacer system according to any one of the preceding claims, **characterised in that** each screw connection (5, 6, 12, 22, 35, 36) and each screw-locking device (8, 18, 28, 38, 58, 68, 74) consist of a biocompatible material.

10. The articular spacer system according to any one of the preceding claims, **characterised in that** spacers are provided between the modules (1, 2, 20, 51, 52, 71, 72) in the form of tooth lock washers with teeth on both sides or wedge locks with wedges on both sides, wherein the spacers are preferably formed from a metal with a lower Vickers hardness than the screw connection (5, 6, 12, 22, 35, 36).

11. The articular spacer system according to any one of the preceding claims, **characterised in that** the articular spacer system is a hip spacer system and comprises three modules (1, 2, 20, 51, 52, 71, 72), wherein the third module (20) is an adapter module (20), which, in an assembled state, connects the first module (1) and the second module (2) at a distance from each other and in a non-detachable manner.

12. The articular spacer system according to claim 11, **characterised in that** the adapter module (20) is angled at an angle that is between 110° and 145°.

13. The articular spacer system according to any one of the preceding claims, **characterised in that** outer surfaces of the modules (1, 2, 20, 51, 52, 71, 72), in an assembled state, are formed by a plastics material (10, 16, 26, 60, 66), in particular by a polymethyl methacrylate bone cement (10, 16, 26, 60, 66), wherein at least one anti-infective agent and/or antiseptic agent are/is suspended and/or dissolved in the plastics material (10, 16, 26, 60, 66), and wherein the threads (5, 6, 35, 36) of the screw connections (5, 6, 12, 22, 35, 36) and of the screw-locking devices (8, 18, 28, 38, 58, 68, 74) are not formed by the plastics material (10, 16, 26, 60, 66), but preferably consist of metal.

14. The articular spacer system according to any one of the preceding claims, **characterised in that** the articular spacer system comprises a multitude of different modules (1, 2, 20, 51, 52, 71, 72) having the same function but different external shapes, such that a multitude of different articular spacers can be assembled from the articular spacer system.

15. A method for assembling an articular spacer using an articular spacer system according to any one of the preceding claims, **characterised in that** the articular spacer is screwed together from at least two modules (1, 2, 20, 51, 52, 71, 72), which are selected from a multitude of different modules (1, 2, 20, 51, 52, 71, 72) according to the anatomy and/or treatment situation, wherein the modules (1, 2, 20, 51, 52, 71, 72) are screwed together such that the screw-locking devices (8, 18, 28, 38, 58, 68, 74) prevent the modules (1, 2, 20, 51, 52, 71, 72) from detaching or being unscrewed.

## Revendications

1. Système d'espaceurs d'articulation modulaire pour l'assemblage par vissage d'un espaceur d'articulation qui est prévu pour le remplacement temporaire d'une articulation artificielle, où le système d'espaceurs d'articulation présente au moins deux modules (1, 2, 20, 51, 52, 71, 72) pouvant être reliés par l'intermédiaire d'une liaison par vissage (5, 6, 12, 22, 35, 36), où un premier module (1, 51, 71) du système d'espaceurs d'articulation présente une surface destinée à la formation d'une surface de glissement de l'articulation de l'espaceur d'articulation et un deuxième module (2, 52, 72) du système d'espaceurs d'articulation présente une tige pour la liaison avec un os, où les modules (1, 2, 20, 51, 52, 71, 72) pouvant être reliés par l'intermédiaire de la liaison par vissage (5, 6, 12, 22, 35, 36) présentent au moins un dispositif de sécurité de vis (8, 18, 28, 38, 58, 68, 74), où l'au moins un dispositif de sécurité de vis (8, 18, 28, 38, 58, 68, 74) est relié avec l'un des modules (1, 2, 20, 51, 52, 71, 72) ou est conçu d'un seul tenant avec un module (1, 2, 20, 51, 52, 71, 72), et
où, deux pièces de dispositif de sécurité de vis (8, 18, 28, 38, 58, 68, 74) sont prévues pour chaque liaison par vissage (5, 6, 12, 22, 35, 36), **caractérisé en ce que** les deux pièces de dispositif de sécurité de vissage sont reliées solidement avec les deux modules (1, 2, 20, 51, 52, 71, 72) à relier par l'intermédiaire de la liaison par vissage (5, 6, 12, 22, 35, 36) ou sont conçus d'un seul tenant avec les deux modules (1, 2, 20, 51, 52, 71, 72) à relier par l'intermédiaire de la liaison par vissage (5, 6, 12, 22, 35, 36) et que les deux pièces de dispositif de sécurité de vis s'interpénètrent de telle façon lors de l'assemblage par vissage des modules (1, 2, 20, 51, 52, 71, 72) qu'elles s'opposent à une libération de la liaison par vissage (5, 6, 12, 22, 35, 36), de préférence, qu'elles empêchent une libération de la liaison par vissage (5, 6, 12, 22, 35, 36).

2. Système d'espaceurs d'articulation selon la revendication 1, **caractérisé en ce que**
le système d'espaceurs d'articulation présente au moins trois modules (1, 2, 20, 51, 52, 71, 72) pouvant être reliés par l'intermédiaire de liaisons par vissage (5, 6, 12, 22, 35, 36) et au moins deux modules (1, 2, 20, 51, 52, 71, 72) présentent au moins un dispositif de sécurité de vis (8, 18, 28, 38, 58, 68, 74).

3. Système d'espaceurs d'articulation selon l'une des revendications précédentes, **caractérisé en ce que**
chaque liaison par vissage (5, 6, 12, 22, 35, 36) présente un filetage intérieur (12, 22) et un filetage extérieur (6, 36) correspondant au filetage intérieur (12, 22), et deux modules (1, 20, 20, 51, 52, 71, 72) pouvant être reliés ensemble par l'intermédiaire d'une liaison par vissage (5, 6, 12, 22, 35, 36) aux extrémités qui se font face l'une à l'autre à l'état relié présentent respectivement le filetage intérieur (12, 22) et le filetage extérieur (6, 36) de cette liaison par vissage (5, 6, 12, 22, 35, 36), de sorte que la liaison par vissage (5, 6, 12, 22, 35, 36) est une partie des deux modules (1, 2, 20, 51, 52, 71, 72).

4. Système d'espaceurs d'articulation selon la revendication 3, **caractérisé en ce que**,
respectivement, au niveau d'un des modules (1, 2, 20, 51, 52, 71, 72) pouvant être reliés ensemble, le dispositif de sécurité de vis (8, 18, 28, 38, 58, 68, 74) est prévu à l'extrémité du filetage extérieur (6, 36) ou au début du filetage intérieur (12, 22) ou sur les deux modules (1, 2, 20, 51, 52, 71, 72) pouvant être reliés ensemble, une pièce de dispositif de sécurité de vis (8, 18, 28, 38, 58, 68, 74) est prévue à l'extrémité du filetage extérieur (6, 36) ou au début du filetage intérieur (12, 22).

5. Système d'espaceurs d'articulation selon l'une des revendications 3 à 4, **caractérisé en ce que**
les modules (1, 2, 20, 51, 52, 71, 72) présentent chacun un noyau métallique (4, 14, 24, 54, 64, 76, 78), où le noyau métallique (4, 14, 24, 54, 64, 76, 78) dépasse hors des extrémités se faisant face des modules (1, 20, 51, 52, 71, 72) à relier ensemble et forme le filetage extérieur (6, 36) à cet endroit et que, dans le noyau métallique (4, 14, 24, 54, 64, 76, 78) de l'autre module (2, 20, 51, 52, 71, 72) parmi les modules (1, 2, 20, 51, 52, 71, 72) à relier ensemble, un trou (19) avec le filetage intérieur (12, 22) est prévu, de sorte que le filetage extérieur (6, 36) et le filetage intérieur (12, 22) forment une liaison par vissage (5, 6, 12, 22, 35, 36).

6. Système d'espaceurs d'articulation selon l'une des revendications précédentes, **caractérisé en ce qu'**également le dispositif de sécurité de vis (8, 18, 28, 38, 58, 68, 74) est formé par au moins un des noyaux métalliques (4, 14, 24, 54, 64, 76, 78), de préférence, le dispositif de sécurité de vis (8, 18, 28, 38, 58, 68, 74) est formé par les deux noyaux métalliques (4, 14, 24, 54, 64, 76, 78).

7. Système d'espaceurs d'articulation selon l'une des revendications précédentes, **caractérisé en ce que**
l'au moins un dispositif de sécurité de vis (8, 18, 28, 38, 58, 68, 74) est constitué de deux disques dentés (74) ou un dispositif de sécurité en coin en deux parties (8, 18, 28, 38, 58, 68).

8. Système d'espaceurs d'articulation selon la revendication 7, **caractérisé en ce que**
les flancs des dents (74) des disques dentés (74) ou des coins (8, 18, 28, 38, 58, 68) des dispositifs de sécurité en coin (8, 18, 28, 38, 58, 68) sont inclinés contre la direction de rotation des éléments de l'au moins une liaison par vissage (5, 6, 12, 22, 35, 36) et la hauteur des dents (74) des disques dentés (74) ou des coins (8, 18, 28, 38, 58, 68) des dispositifs de sécurité en coin (8, 18, 28, 38, 58, 68) est supérieure à la hauteur des pas des filetages (5, 6, 35, 36) des éléments de l'au moins une liaison par vissage (5, 6, 12, 22, 35, 36).

9. Système d'espaceurs d'articulation selon l'une des revendications précédentes, **caractérisé en ce que**
chaque liaison par vissage (5, 6, 12, 22, 35, 36) et chaque dispositif de sécurité de vis (8, 18, 28, 38, 58, 68, 74) est constitué par un matériau biocompatible.

10. Système d'espaceurs d'articulation selon l'une des revendications précédentes, **caractérisé en ce qu'**entre
les modules (1, 2, 20, 51, 52, 71, 72), des espaceurs sous la forme de disques dentés avec une dentition bilatérale ou des dispositifs de sécurité en coin avec des coins bilatéraux sont prévus, où, de préférence, les espaceurs sont constitués d'un métal avec une dureté de Vickers faible servant de liaison par vissage (5, 6, 12, 22, 35, 36).

11. Système d'espaceurs d'articulation selon l'une des revendications précédentes, **caractérisé en ce que**
le système d'espaceurs d'articulation est un système d'espaceur pour hanche et présente trois modules (1, 2, 20, 51, 52, 71, 72), où le troisième module (20) est un module adaptateur (20) qui, dans l'état assemblé, sépare le premier module (1) du deuxième module (2) et les fixe l'un à l'autre de manière inamovible.

12. Système d'espaceurs d'articulation selon la revendication 11, **caractérisé en ce que**
le module adaptateur (20) est anguleux et présente un angle entre 110 ° et 145 °.

13. Système d'espaceurs d'articulation selon l'une des revendications précédentes, **caractérisé en ce que**
les surfaces extérieures des modules (1, 2, 20, 51, 52, 71, 72) à l'état assemblé sont formées par une matière plastique (10, 16, 26, 60, 66), sont notamment formées par un ciment osseux de polyméthylméthacrylate (10, 16, 26, 60, 66), où, au moins un anti-infectieux et/ou un antiseptique est, ou sont, en suspension et/ou est, ou sont, dissout dans la matière plastique (10, 16, 26, 60, 66) et où les filetages (5, 6, 35, 36) des liaisons par vissages (5, 6, 12, 22, 35, 36) et les dispositifs de sécurité de vis (8, 18, 28, 38, 58, 68, 74) ne sont pas formés par la matière plastique (10, 16, 26, 60, 66), mais sont de préférence constitués de métal.

14. Système d'espaceurs d'articulation selon l'une des revendications précédentes, **caractérisé en ce que**
le système d'espaceurs d'articulation présente un grand nombre de modules (1, 2, 20, 51, 52, 71, 72) différents, de même fonction, mais de forme extérieure différente, de sorte que l'on peut assembler un grand nombre d'espaceurs d'articulation différents à partir du système d'espaceurs d'articulation.

15. Procédé d'élaboration d'un espaceur d'articulation avec un système d'espaceurs d'articulation selon l'une des revendications précédentes, **caractérisé en ce que**
l'espaceur d'articulation est assemblé par vissage à partir d'au moins deux modules (1, 2, 20, 51, 52, 71, 72) qui sont choisis en fonction de l'anatomie et/ou de la situation de traitement parmi un grand nombre de modules (1, 2, 20, 51, 52, 71, 72) différents, où les modules (1, 2, 20, 51, 52, 71, 72) sont vissés ensemble de telle manière que les dispositifs de sécurité de vis (8, 18, 28, 38, 58, 68, 74) empêchent une libération ou un dévissage des modules (1, 2, 20, 51, 52, 71, 72).
